# EUROPEAN PATENT SPECIFICATION

(11) **EP 1 318 849 B1**
(45) Date of publication and mention of the grant of the patent: **29.04.2009**
(21) Application number: 01967315.1
(22) Date of filing: 07.09.2001
(51) Int. Cl.: A61M 15/00

(54) **INHALATION DEVICE**
INHALIERVORRICHTUNG
DISPOSITIF INHALATEUR

(30) Priority: 19.09.2000 AU PR020400
(43) Date of publication of application: 18.06.2003
(73) Proprietor: GLAXO GROUP LIMITED, Greenford, Middlesex UB6 ONN (GB)
(72) Inventor: PIKE, Gregory, Charles c/o Reel Magic Pty Ltd, Dandenong, VIC 3175 (AU)
(74) Representative: Rice, Jason Neale
(86) International application number: PCT/EP2001/010327
(87) International publication number: WO 2002/024263

(56) References cited:
- WO-A-01/72605
- US-A- 5 447 151
- US-A- 5 622 166
- US-A- 5 823 178
- US-A- 6 032 666

## Description

### Technical Field

The present invention relates to an inhalation device and more particularly to an inhalation device for delivering doses of powdered medicament

### Background to the Invention

Many forms of inhalation devices currently available for delivering metered doses of powdered medicament are designed to contain multiple doses. Although inhalation devices containing multiple doses adequately fulfil certain market requirements, they are not necessarily applicable to developing countries or poorer communities in which consumers may, at any one time, only be able to afford the cost of a few medicament doses or even a single medicament dose.

US-A-5 447 151 discloses a cartridge according to the pre-characterising part of claim 1, further background art is disclosed in US-A-6 032 666 and US-A-5 622 166.

### Summary of the Invention

According to the present invention there is provided a cartridge according to claim 1.

The present invention further provides an inhalation device according to claim 5.

Particularly advantageousty, the airflow induced by inhalation by the user represents the entirety of the air flow for delivery of the unit dose, without additional assistance by pump means or the like. Accordingly it is particularly desirable for the inhalation device to incorporate indicator means to indicate to the user that an adequate inhalation-induced air flow has been achieved to effect delivery of the complete unit dose to the user. In one form, this indicator means may comprise a member displaceable in response to a minimum air flow rate. Conveniently, such an indicator member may be in the form of a rotatable vane mounted within the air flow path through the device and subject to a predetermined resistance whereby an inhalation-induced air flow sufficient to cause delivery of a unit dose is able to overcome the resistance and cause the vane to spin or rotate, with such spinning motion being observable by a user. The resistance required to be overcome to induce spinning motion can either be a direct frictional resistance to vane rotation and/or a resistance provided in an air flow valve opened in response to adequate inhalation-induced air flow.

Preferably, the indicator means is visible to the user.

Particularly advantageously, the cartridge has a body of disc-like form, with the compartments being arranged around the body. The body is indexable to present successive compartments at a delivery position for discharge of unit doses from those compartments.

In one embodiment, the separator is located in the delivery position and indexing rotation of the disc-like body of the cartridge relative to the separator causes the separator to peel the sealing layer from the compartment moving into the delivery position by actuation of an indexing mechanism.

Preferably, the indexing mechanism comprises an actuating member carried by the casing and movable between a first position which represents an inactive state of the inhalation device in which, preferably, the inhalation aperture is closed, and a second position in which the cartridge body is caused to rotate to present a subsequent compartment to the delivery position in which the inhalation aperture is opened.

Although the cartridge may be supplied to a user with all available compartments filled with medicament, it is possible for only one or a few such compartments to contain medicament sufficient, for example, for a single use or a single day's use.

Particularly advantageously, the cartridge can be removed and replaced without the need for depletion of all of the unit doses within the cartridge. Preferably, insertion or removal of the cartridge into or from the casing does not affect peeling of the sealing layer. More preferably, insertion or removal of the cartridge into or from the casing cannot be effected when the inhalation aperture is in the open position.

In a particularly preferred embodiment, the cartridge comprise a disc-like body with said compartments being arranged in angularly spaced array around the body, and a disc-like layer on said body to seal said compartments, the separator member being interposed between the body and the sealing layer, and extending substantially radially of said body. The body is rotatable relative to the separator to cause the sealing layer to peel to open successive compartments when the body is rotated stepwise relative to the separator.

In one particular preferred form, inhalation-induced air flow from one end of the channel of the separator to the other entrains the unit dose within the compartment aligned with the separator for delivery through the inhalation aperture.

Preferably, the configuration is such that when the cartridge is mounted within the casing the inhalation aperture lies immediately adjacent the radially outer end of the air flow passage in the separator.

### Brief Description of the Drawings

An embodiment of the invention will now be described, by way of example only, with reference to the accompanying drawings, in which:
Figure 1 is a perspective view of an inhalation device according to the present invention showing a casing of the device in an open state;

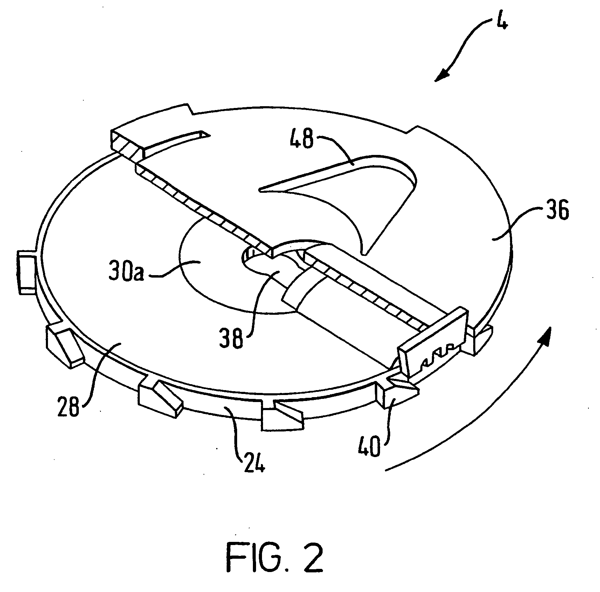

Figure 2 is a perspective view, partly cut away, showing the cartridge;
Figure 3 is an exploded view of the cartridge showing its major component parts;
Figure 4 is a perspective view showing the cartridge mounted within the casing, the view being partially cut-away and in partially schematic form with a peelable sealing layer of the cartridge omitted to better illustrate an inhalation-induced air flow path through the casing and through an open compartment of the cartridge; and
Figure 5 is a schematic view illustrating the casing in its closed state.

### Detailed Description of the Drawings

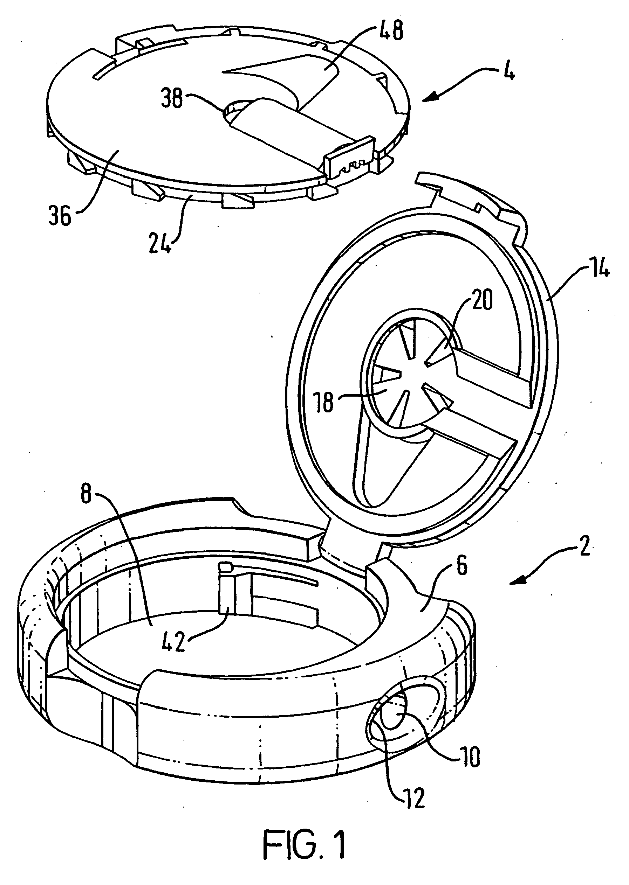

Figure 1 shows that the inhalation device of the preferred embodiment comprises a casing 2 adapted to receive a removable/replaceable cartridge 4 containing one or more unit doses of powdered medicament. The casing 2 comprises a main body 6 with an internal chamber 8 for receiving the cartridge 4 which is of disc-like form and which will be described in greater detail subsequently. The cartridge 4 is mounted within the chamber 8 for indexing rotation about the axis of the cartridge in order to present successive ones of the unit doses at a delivery position in the chamber 8. The main body 6 also has in its peripheral wall an inhalation aperture 10 through which a unit dose of the medicament at the delivery position can be withdrawn from the cartridge 4 under the effect of inhalation of the user, the configuration of the body 6 in that part of the casing being such that it forms a mouthpiece 12 which can be placed to the mouth of a user. The chamber 8 with the cartridge 4 therein is closeable by means of a lid 14 carried by the body 6. In the embodiment shown, the lid 14 is pivotally mounted to the body 6, although in other embodiments alternative mounting arrangements such as a slidable mounting for the lid could be adopted.

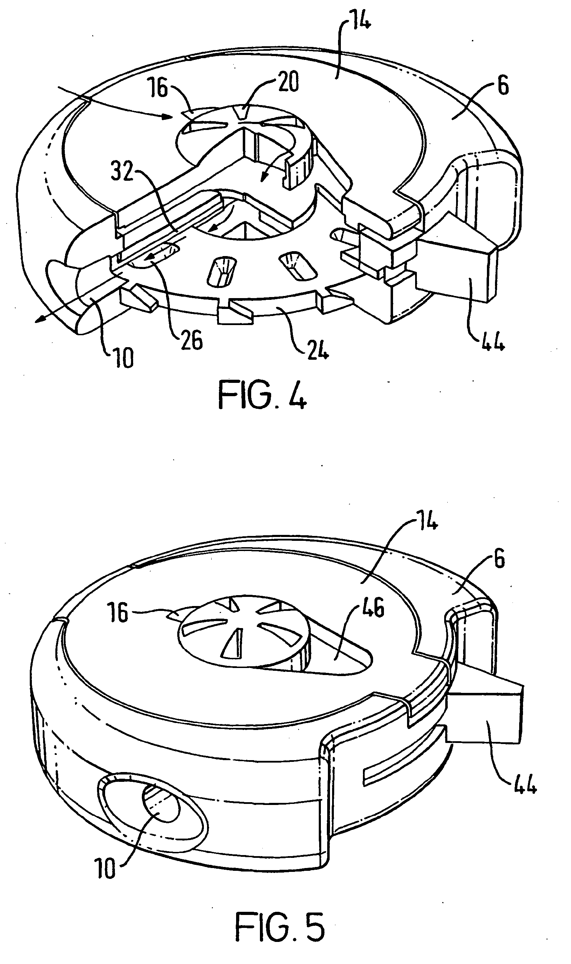

The lid 14 incorporates an inlet port 16 (see Figures 4 & 5) which provides an air inlet into the chamber 8 when the chamber 8 is closed by the lid 14. The inlet port 16 communicates with the interior of the chamber 8 via a cavity (not shown) within the lid 4, the cavity housing air flow indicator means. In the embodiment shown, the indicator means is in the form of a rotatable indicator vane 20 which can be put into rotation by the incoming air flow when the air flow rate exceeds a predetermined minimum which is that which is sufficient to ensure take-up of a complete unit dose from the cartridge. This effect may be achieved by incorporating a certain frictional resistance in the rotational mounting of the vane 20 which is determined empirically to permit vane rotation only when the required minimum air flow rate is achieved. An air outlet from the cavity (not shown) into the chamber 8 may be closed by a one-way flexible flap valve (not shown) which opens in response to incoming air flow; the effect of this valve may be such that the indicator vane 20 may be freely rotatable in response to any significant air flow sufficient to open the valve thereby obviating the need to incorporate a frictional resistance into the mounting for the vane. The cavity (not shown) in the lid 14 is closed by a transparent cover so that a user can readily see rotation of the vane 20 thereby indicating that the air flow caused by the inhalation is sufficient to deliver the unit dose. In this regard it is to be understood that in the device of this embodiment, to reduce the cost of manufacture, delivery of the unit dose is effected just by the air flow induced by inhalation of the user and without incorporating any additional means such as pumps or turbines for providing a forced air flow.

The cartridge 4 comprises a disc-like base or body 24 formed with a series of compartments in the form of pockets 26 distributed around the axis of the cartridge with each pocket 26 being of elongate form extending substantially radially of the body 24 and opening onto a substantially planar surface 24a of the body 24 (the upper surface as shown in the drawings). The body 24a as illustrated has ten such pockets 26 each adapted to contain a unit dose, although in other forms the body may have fewer than ten pockets or, where space requirements permit, more than ten pockets. It is however to be understood that although the basic cartridge as illustrated is designed to contain ten unit doses, on unit dose per pocket 26, it is envisaged that the cartridge can be supplied with only one, or a few, of its pockets 26 filled with unit doses of medicament thereby enabling a customer with limited financial resources to purchase a cartridge having medicament suitable for, for example, a single day's requirements. The pockets 26 are closed by a peelable layer 28, for example a foil layer, applied to the surface 24a of the cartridge body 24.

As shown in Figure 4, the cartridge 4 also includes a separator 30 interposed between the body 24 and the layer 28 and extending radially from an integral hub portion 30a located within a central aperture of the cartridge body 24 and defining a central hub about which the body 24 and layer 28 attached thereto can rotate relative to the separator 30. The surface of the separator 30 in contact with the surface 24a of the cartridge body 24 is formed with an air flow channel 32 which extends radially from the hub portion 30a to the exterior of the cartridge body to communicate with the inhalation aperture 10. As will subsequently be described, relative movement between the body 24 of the cartridge and the separator 30 will cause the separator 30 to progressively peel the layer 28 away from the surface 24a of the cartridge body 24 and thereby open the pockets 26 successively. When the separator 30 is in substantial radial alignment with a pocket 26 the air flow channel 32 of the separator 30 will extend along the open side of that pocket 26 so that an inhalation-induced air flow passing through the channel 32 of the separator 30 and the adjacent part of the pocket 26 will entrain medicament within that pocket for discharge through the aperture 10. The air flow effect induced through the open pocket tends to cause deagglomeration of smaller particles from coarser particles within the pocket thereby ensuring effective discharge of the entire dose, provided the induced air flow is adequate. The separator 30 may contain a mesh, grid, or baffles integrally moulded therein to assist deagglomeration of the particles.

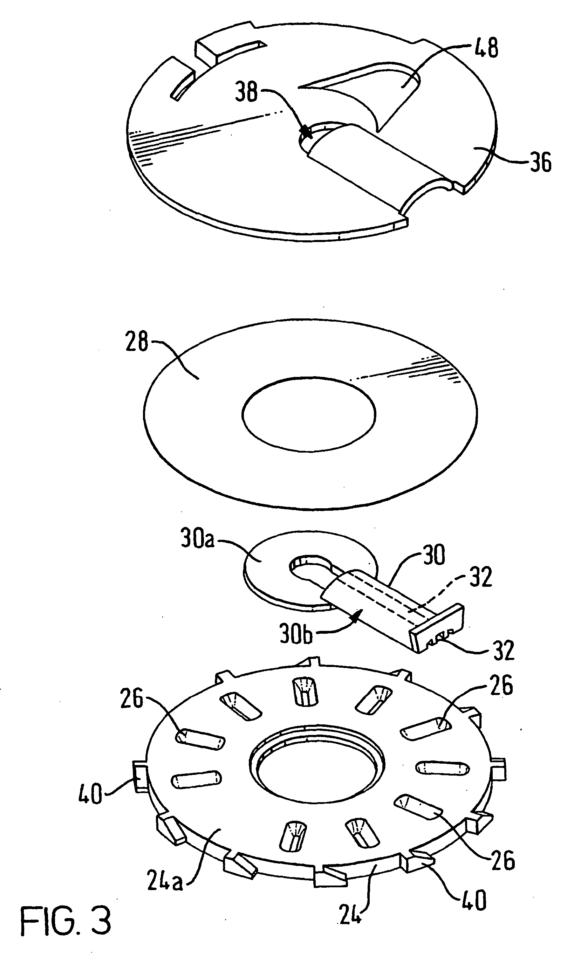

To facilitate effective separation of the layer 28 and cartridge body 24 in a controlled manner, the outer surface of the separator 30 is appropriately profiled at least at its leading edge portion with reference to the direction of rotation of the cartridge body. As shown in Figure 3, that profile is a rounded profile (see 30b) although a more wedge-shaped profile could alternatively be adopted.

The cartridge also includes a cover 36 which overlies the upper surface of the layer 28 to protect the layer form damage. The cover 36 remains stationary in position relative to the separator 30 and includes an air inlet aperture 38 which lies adjacent to the outlet of the indicator vane cavity 18 in the lid with the lid 14 closed so that inhalation-induced air flow enters the chamber 8 via the indicator vane cavity 18 in the lid 14 and passes via the inlet aperture 38 in the cartridge cover 36 into the radially inner end of the air flow channel 32 in the separator 30. However, when the cartridge is supplied with only one or two of its pockets filled with unit doses, the cover can be omitted.

The body 24 of the cartridge has, around its periphery, a series of ramped ratchet teeth 40 arranged to co-operate with an indexing member mounted in the wall of the main body 6 of the casing 2, the indexing member having an inwardly projecting ratchet tooth 42 to engage successive ones of the teeth 40 and an externally-projecting actuating knob 44 which is slidable to move the indexing member backwards and forwards over a predetermined arc of rotation. The co-operation between the indexing member and the ratchet teeth 40 is such that manual actuation of the indexing member by a user will advance the cartridge body 24 in a single direction of rotation through an incremental step which corresponds to angular distance between two adjacent pockets 26 in the cartridge body. The indexing member may also include a shutter (not shown) associated with the inhalation aperture 10 whereby reciprocation of the indexing member by the knob 44 also causes the shutter to open and close the inhalation aperture 10. In one end position of the actuating knob 44 which represents an inactive state of the inhalation device, the inhalation aperture 10 is closed by the shutter; movement of the knob 44 to its other end position places the device in an active state by opening the inhalation aperture 10 and also by displacing the body 24 of the cartridge through one step. The shutter may also contain a mesh or grid integrally moulded therein to assist deagglomeration of particles.

The cartridge 4 and the chamber 8 of the casing 2 are so configured that the cartridge 4 can only be fitted into the chamber 8 in a predetermined angular orientation in which the outer end of the separator 30 is in alignment with the inhalation aperture 10 (this being the delivery position) and the inlet aperture 38 in the cartridge cover 36 is in alignment with the air outlet from the indicator vane cavity (not shown). Also in this position, a sight window 46 (preferably having a magnifying effect) in the lid 14 is in alignment with an aperture 48 in the cartridge cover 36. Thus a user can view a number printed on the layer 28 underlying the aperture 48 and indicating the number of doses remaining in the cartridge or the number of doses taken (as determined by the angular position of the cartridge body 24 relative to the separator 30). The aperture 48 in the cartridge cover also allows viewing of other indicia on the cartridge.

When a cartridge is placed into the casing and the lid closed, displacement of the actuating knob 44 from one end position (the end portion corresponding to the inactive state) into the other end position will cause opening of the shutter and will also cause the body of the cartridge to index through one incremental step. This in turn places the first pocket (in the case of a new cartridge) in alignment with the air flow channel 32 of the separator 30 to thereby enable delivery of the unit dose from the pocket, with the separator 30 peeling away the layer from the pocket during the movement. The lips of the user are then placed over the mouthpiece 12 of the casing body and the user inhales through the inhalation aperture in order to draw air through the chamber 8 via the open pocket aligned with the separator 30, the vane 20 spinning to indicate to the user that the air flow has been sufficient to enable the correct dose to be taken. The actuating knob 44 is then returned to its starting position and this also causes the shutter to close the inhalation aperture 10.

A cartridge can be removed and replaced into the casing as required; removal of the cartridge does not require all of the unit doses to have been dispensed. Insertion of a cartridge into the casing does not itself open a pocket, this action only occurring when the actuating knob 44 is displaced to cause indexing of the cartridge body and hence peeling of the foil layer; preferably, the locating system between the cartridge and the chamber is such that insertion and removal of cartridges can only be effected when the actuating knob 44 is in its end position in which the inhalation aperture is closed.

Only a single dose can be inhaled in each operation, inhalation of a second dose requiring positive movement of the actuating knob through a complete cycle. Each unit dose is delivered in a discrete action and if during that action the user happens to exhale, this will not affect the successful delivery of subsequent doses; loss of the current dose, if the user exhales during delivery, can be prevented by the incorporation of the one-way valve at the air outlet from the cavity. The presence of the cartridge cover protects the user form exposure to residue within previously opened cartridges in the event that the induced air flow was insufficient to fully discharge the dose.

The configuration of the cartridge and of the casing enables the inhalation device to be stored for use in any orientation without adversely affecting the efficiency of subsequent discharge of a unit dose.

Although the construction of the casing and cartridge provide benefits for use in developing countries and poorer communities as discussed above, the overall design is very compact and can fit easily into a purse or small handbag, whereby the device can be used by anyone who does not wish to carry conventional devices which tend to be rather bulky.

The inhalation device of the invention is suitable for dispensing medicament, particularly for the treatment of respiratory disorders. Appropriate medicaments may thus be selected from, for example, analgesics, e.g., codeine, dihydromorphine, ergotamine, fentanyl or morphine; anginal preparations, e.g., diltiazem; antiallergics, e.g., cromoglycate (e.g. s the sodium salt), ketotifen or nedocromil (e.g. as the sodium salt); antiinfectives e.g., cephalosporins, penicillins, streptomycin, sulphonamides, tetracyclines and pentamidine; antihistamines, e.g., methapyrilene; anti- inflammatories, e.g., beclomethasone (eg as the dipropionate ester), fluticasone (e.g. as the propionate ester), flunisolide, budesonide, rofleponide, mometasone e.g. as the furoate ester), ciclesonide, triamcinolone (e.g. as the acetonide) or 6α, 9α-difluoro-11β-hydroxy-16α-methyl-3-oxo-17α-propionyloxy-androsta-1,4-diene-17β-carbothioic acid S-(2-oxo-tetrahydro-furan-3-yl) ester; antitussives, e.g., noscapine; bronchodilators, e.g., albuterol (eg as free base or sulphate), salmeterol (eg as xinafoate), ephedrine, adrenaline, fenoterol (eg as hydrobromide), formoterol (eg as fumarate), isoprenaline, metaproterenol, phenylephrine, phenylpropanolamine, pirbuterol (eg as acetate), reproterol (eg as hydrochloride), rimiterol, terbutaline (eg as sulphate), isoetharine, tulobuterol or 4-hydroxy-7-[2-[[2-[[3-(2-phenylethoxy)propyl]sulfonyl]ethyl]amino]ethyl-2(3H)-benzothiazolone; adenosine 2a agonists, eg 2R,3R,4S,5R)-2-[6-Amino-2-(1S-hydroxymethyl-2-phenyl-ethylamino)-purin-9-yl]-5-(2-ethyl-2H-tetrazol-5-yl)-tetrahydro-furan-3,4-diol (e.g. as maleate); α4 integrin inhibitors eg (2S)-3-[4-({[4-(aminocarbonyl)-1-piperidinyl]carbonyl}oxy)phenyl]-2-[((2S)-4-methyl-2-{[2-(2-methylphenoxy) acetyl]amino}pentanoyl)amino] propanoic acid (e.g as free acid or potassium salt), diuretics, e.g., amiloride; anticholinergics, e.g., ipratropium (eg as bromide), tiotropium, atropine or oxitropium; hormones, e.g., cortisone, hydrocortisone or prednisolone; xanthines, e.g., aminophylline, choline theophyllinate, lysine theophyllinate or theophylline; therapeutic proteins and peptides, e.g., insulin or glucagon; vaccines, diagnostics, and gene therapies. It will be clear to a person skilled in the art that, where appropriate, the medicaments may be used in the form of salts, (e.g., as alkali metal or amine salts or as acid addition salts) or as esters (e.g., lower alkyl esters) or as solvates (e.g., hydrates) to optimise the activity and/or stability of the medicament.

Preferred medicaments are selected from albuterol, salmeterol, fluticasone propionate and beclomethasone dipropionate and salts or solvates thereof, e.g., the sulphate of albuterol and the xinafoate of salmeterol.

Medicaments can also be delivered in combinations. Preferred formulations containing combinations of active ingredients contain salbutamol (e.g., as the free base or the sulphate salt) or salmeterol (e.g., as the xinafoate salt) or formoterol (eg as the fumarate salt) in combination with an anti-inflammatory steroid such as a beclomethasone ester (e.g., the dipropionate) or a fluticasone ester (e.g., the propionate) or budesonide. A particularly preferred combination is a combination of fluticasone propionate and salmeterol, or a salt thereof (particularly the xinafoate salt). A further combination of particular interest is budesonide and formoterol (e.g. as the fumarate salt). A further combination of particular interest is salmeterol, or a salt thereof (particularly the xinafoate salt) and ipratropium bromide.

It will be understood that the present disclosure is for the purpose of illustration only and the invention extends to modifications, variations and improvements thereto within the scope of the appended claims.

## Claims

1. A cartridge (4), for containing unit doses of powdered medicament therein, the cartridge adapted for removable incorporation into an inhalation device and comprising a body (24), a plurality of compartments (26) within said body each for containing a discrete unit dose of powdered medicament, and a sealing layer (28) applied to the body for closing each said compartment;
**characterised by**:
the cartridge comprises
a separator (30) for removing said sealing layer (28) to open each compartment (26) and thereby define an air flow passage via said open compartment, the separator (30).
being mounted in the cartridge (4) such that the body is movable relative thereto, and
comprising a member interposed between the body (24) and the seating layer and extending substantially radially of the body which:
removes said seating layer upon movement of the body relative to the separator; and
incorporates a channel (32) which defines the air flow passage
across an opened compartment (26) when the compartment is aligned with the separator member.

2. A cartridge according to claim 1, wherein the body (24) is disc-like with the compartments being arranged in an angularly spaced array around the body.

3. A cartridge according to claim 1 or 2, wherein the body (24) of the cartridge (4) is rotatable relative to the separator (30) to cause the sealing layer (28) to peel and open successive compartments (26) when the body is rotated stepwise relative to the separator.

4. A cartridge according to any one of claims 1 to 3, wherein the cartridge (4) contains at least one unit dose of powdered medicament therein.

5. An inhalation device for delivering unit doses of powdered medicament, said device comprising:
a casing (2), and
a cartridge (4) according to any one of claims 1 to 4 removably mounted within said casing;
wherein the casing (2) has an inhalation aperture (10) whereby inhalation of air by a user causes air to be drawn via said air flow passage to entrain within the air flow the contents of an opened compartment (26) aligned with the separator member.

6. An inhalation device according to claim 5, additionally comprising indicator means (20) to indicate that an air flow has been achieved to effect delivery of the complete unit dose to the user.

7. An inhalation device according to claim 5 or 6, additionally comprising an indexing mechanism to present successive compartments (26) in the cartridge (4) to a delivery position for discharge of unit doses from each compartment.

8. An inhalation device according to claim 7, wherein the indexing mechanism comprises an actuating member (44) in the casing movable between a first position in which the inhalation aperture (10) is closed, and a second position wherein a subsequent compartment (26) is moved to the delivery position and the inhalation aperture (10) is open.

9. An inhalation device according to any one of claims 5 to 8, wherein the inhalation aperture (10) lies immediately adjacent the radially outer end of the air flow passage defined by the separator member.

## Patentansprüche

1. Patrone (4) zum Aufnehmen von Stückdosen eines pulvrigen Medikaments darin, wobei die Patrone zum entfernbaren Einführen in ein Inhalationsgerät eingerichtet ist, und umfasst: einen Körper (24), eine Vielzahl von Fächern (26) innerhalb des Körpers, jedes zum Aufnehmen einer diskreten Stückdosis eines pulvrigen Medikaments, und eine am Körper zum Schließen jedes Fach angebrachte Abdichtschicht (28),
**gekennzeichnet dadurch, dass**
die Patrone eine Trenneinrichtung (30) zum Entfernen der Abdichtschicht (28) umfasst, um jedes Fach (26) zu öffnen und **dadurch** einen Luftstromdurchgang über das offene Fach zu definieren, wobei die Trenneinrichtung (30)
in der Patrone (4) derart befestigt ist, dass der Körper relativ hierzu beweglich ist, und
ein zwischen dem Körper (24) und der Abdichtschicht eingefügtes und sich im Wesentlichen radial vom Körper erstreckendes Element umfasst, das:
die Abdichtschicht auf Bewegung des Körpers relativ zur Trenneinrichtung entfernt, und
einen Kanal (32) einführt, der den Luftstromdurchgang über ein geöffnetes Fach (26) definiert, wenn das Fach mit dem Trenneinrichtungselement ausgerichtet ist.

2. Patrone gemäß Anspruch 1, bei welcher der Körper scheibenförmig ist, wobei die Fächer in einer winkelmäßig beabstandeten Ordnung um den Körper angeordnet sind.

3. Patrone gemäß Anspruch 1 oder 2, bei welcher der Körper (24) der Patrone (4) relativ zur Trenneinrichtung (30) drehbar ist, um die Abdichtschicht (28) nachfolgende Fächer (26) lösen und öffnen zu lassen, wenn der Körper schrittweise relativ zur Trenneinrichtung gedreht wird.

4. Patrone gemäß einem der Ansprüche 1 bis 3, bei welcher die Patrone (4) zumindest eine Stückdosis des pulvrigen Medikaments darin aufnimmt.

5. Inhalationsgerät zum Zuführen von Stückdosen eines pulvrigen Medikaments, wobei die Vorrichtung umfasst:
ein Gehäuse (2), und
eine Patrone gemäß einem der Ansprüche 1 bis 4, die entfernbar im Gehäuse befestigt ist,
wobei das Gehäuse (2) einen Inhalationsdurchlass (10) aufweist, wodurch eine Luftinhalation durch einen Benutzer Luft über den Luftstromdurchgang ziehen lässt, um mit dem Luftstrom die Inhalte eines mit der Trenneinrichtung ausgerichteten, geöffneten Fachs (26) mitzunehmen.

6. Inhalationsgerät gemäß Anspruch 5, das zusätzlich ein Anzeigemittel (20) umfasst, um anzuzeigen, dass ein Luftstrom erreicht wurde, um ein Zuführen der kompletten Stückdosis zum Benutzer zu bewirken.

7. Inhalationsgerät gemäß Anspruch 5 oder 6, das zusätzlich einen Indizierungsmechanismus umfasst, um nachfolgende Fächer (26) in der Patrone (4) in eine Zuführposition zum Ausstoßen von Stückdosen von jedem Fach darzubieten.

8. Inhalationsgerät gemäß Anspruch 7, bei dem der Indizierungsmechanismus ein Betätigungsbauteil (44) im Gehäuse umfasst, das zwischen einer ersten Position, in welcher der Inhalationsdurchlass (10) geschlossen ist, und einer zweiten Position, bei der ein nachfolgendes Fach (26) in eine Zuführposition bewegt wird und der Inhalationsdurchlass (10) geöffnet ist, beweglich ist.

9. Inhalationsgerät gemäß einem der Ansprüche 5 bis 8, bei dem der Inhalationsdurchlass (10) unmittelbar benachbart zum radial äußeren Ende des durch die Trenneinrichtung definierten Luftstromdurchgangs liegt.

## Revendications

1. Cartouche (4) pour contenir des doses unitaires de médicament en poudre, la cartouche étant adaptée pour être incorporée de manière amovible dans un dispositif d'inhalation et comprenant un corps (24), une pluralité de compartiments (26) à l'intérieur dudit corps, chacun pour contenir une dose unitaire distincte de médicament en poudre, et une couche d'étanchéité (28) appliquée sur le corps pour fermer chacun desdits compartiments ;
**caractérisée en ce que** la cartouche comprend :
un séparateur (30) pour retirer ladite couche d'étanchéité (28) pour ouvrir chaque compartiment (26) et définir ainsi un passage d'air via ledit compartiment ouvert, le séparateur (30) :
étant monté dans la cartouche (4) de sorte que le corps est mobile par rapport à celle-ci, et
comprenant un élément intercalé entre le corps (24) et la couche d'étanchéité qui s'étend sensiblement de manière radiale par rapport au corps, qui :
retire ladite couche d'étanchéité suite au mouvement du corps par rapport au séparateur ; et
incorpore un canal (32) qui définit le passage d'air sur un compartiment ouvert (26) lorsque le compartiment est aligné avec l'élément séparateur.

2. Cartouche selon la revendication 1, dans laquelle le corps (24) est en forme de disque avec les compartiments qui sont agencés dans une structure angulairement espacée autour du corps.

3. Cartouche selon la revendication 1 ou 2, dans laquelle le corps (24) de la cartouche (4) tourne par rapport au séparateur (30) pour amener la couche d'étanchéité (28) à se détacher et à ouvrir les compartiments (26) successifs lorsque le corps tourne par palier par rapport au séparateur.

4. Cartouche selon l'une quelconque des revendications 1 à 3, dans laquelle la cartouche (4) contient au moins une dose unitaire de médicament en poudre.

5. Dispositif d'inhalation destiné à administrer des doses unitaires de médicament en poudre, ledit dispositif comprenant :
un boîtier (2) ; et
une cartouche (4) selon l'une quelconque des revendications 1 à 4, montée de manière amovible à l'intérieur dudit boîtier ;
dans lequel le boîtier (2) a une ouverture d'inhalation (10), moyennant quoi l'inhalation de l'air par un utilisateur provoque l'aspiration de l'air via ledit passage d'air afin d'entraîner dans l'écoulement d'air, le contenu d'un compartiment ouvert (26) aligné avec l'élément séparateur.

6. Dispositif d'inhalation selon la revendication 5, comprenant en outre des moyens d'indication (20) pour indiquer qu'un écoulement d'air a été obtenu afin d'administrer la dose unitaire complète à l'utilisateur.

7. Dispositif d'inhalation selon la revendication 5 ou 6, comprenant en outre un mécanisme d'indexation pour présenter des compartiments successifs (26) dans la cartouche (4) dans une position d'administration pour la décharge des doses unitaires de chaque compartiment.

8. Dispositif d'inhalation selon la revendication 7, dans lequel le mécanisme d'indexation comprend un élément d'actionnement (44) dans le boîtier, pouvant être déplacé entre une première position dans laquelle l'ouverture d'inhalation (10) est fermée, et une seconde position dans laquelle un compartiment consécutif (26) est déplacé dans la position d'administration et l'ouverture d'inhalation (10) est ouverte.

9. Dispositif d'inhalation selon l'une quelconque des revendications 5 à 8, dans lequel l'ouverture d'inhalation (10) est immédiatement adjacente à l'extrémité radialement externe du passage d'air défini par l'élément séparateur.
